Europäisches Patentamt

European Patent Office

Office européen des brevets

(11) Publication number: **0 083 129**
**B1**

(12) **EUROPEAN PATENT SPECIFICATION**

(45) Date of publication of patent specification: **05.02.86**

(51) Int. Cl.⁴: **A 61 K 49/02, C 07 B 59/00**

(21) Application number: **82201602.8**

(22) Date of filing: **14.12.82**

(54) **Method of preparing radionuclide-labelled proteins, in particular antibodies or antibody fragments.**

(30) Priority: **29.12.81 NL 8105880**

(43) Date of publication of application:
**06.07.83 Bulletin 83/27**

(45) Publication of the grant of the patent:
**05.02.86 Bulletin 86/06**

(84) Designated Contracting States:
**AT BE CH DE FR GB IT LI NL SE**

(56) References cited:
**EP-A-0 017 355**
**EP-A-0 035 765**
**EP-A-0 038 546**

(73) Proprietor: Mallinckrodt Diagnostica (Holland)
B.V.
Westerduinweg 3
NL-1755 LE Petten (NL)

(72) Inventor: **Goedemans, Wilhelmus Theodorus**
**c/o OCTROOIBUREAU Zoan B.V. Apollolaan 151**
**NL-1077 AR Amsterdam (NL)**

(74) Representative: Swaters, Pieter D. et al
Octrooibureau ZOAN B.V. Apollolaan 151
NL-1077 AR Amsterdam (NL)

EP 0 083 129 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to a method of preparing radionuclide-labelled proteins which are unstable at a pH lower than the physiological pH, in particular antibodies or antibody fragments, by reacting a protein which is provided with chelating groups derived from desferrioxamine, 8-hydroxyquinoline, imino triacetic acid-containing chelating agents, or iminodiacetic acid-containing chelating agents, such as ethylene diamine tetraacetic acid, diethylene triamine pentacetic acid or diaminocyclohexyl tetracetic acid, or salts hereof, or a chelating peptide or polypeptide such as transferrine, with a solution of a compound of a radionuclide, selected from radioactive indium, radioactive gallium, lead-203, ruthenium-97, mercury-197 and thallium (III)-201.

The invention furthermore relates to radionuclide-labelled proteins, in particular antibodies or antibody fragments obtained by using said method, to a radio-pharmaceutical composition comprising said labelled macromolecules, and to a kit suitable for performing a radiodiagnostic examination.

Radionuclide-labelled compounds may be used for diagnostic examination, for example, into deviations in the shape and function of internal organs and into the presence and location of pathological processes in the body. For this purpose, a composition in which the radioactive compound is present, is administered to the patient, for example, in the form of an injectable liquid. By means of suitable detection devices, for example a gamma camera, images can be obtained—by recording the emitted radiation—of, for example, the organ or the pathological process in which the radioactive compound is incorporated (scanning).

Radioactive-labelled biological macromolecules, in particular proteins, present interesting prospects for diagnostic application. Certain proteins have a very great target organ specificity and, after having been introduced into the patient's body, can react very selectively with biological macromolecules present therein; a good example hereof is the selective reaction of antibodies with antigens present in the body.

The direct labelling of a protein with a metallic radionuclide has two disadvantages. First of all, the biologically active place of the protein necessary for a good target organ specificity is easily blocked by said reaction so that the normal behaviour of the biological macromolecule is disturbed. In addition, the affinity between metal ion and macromolecule is often insufficient, as a result of which the bond formed is not stable enough to remain intact under physiological conditions; then it can no longer be determined how the protein behaves in the body.

Various investigators have therefore provided the biological macromolecules, usually a protein, with chelating groups with which the radionuclide can enter into a rigid bond. Of course, the biological behaviour of the original macromolecule must be maintained as good as possible by this modification. For example, Sundberg and cooperators (J. Med. Chem. *17* (1974), 1304—07) have used the coupling product of 1-(p-benzenediazonium)ethylene diamine tetraacetic acid with a protein as a chelating agent for radioactive indium ions and studied the resulting chelate.

Krejcarek c.s. have described in Biochem. Biophys. Res. Commun. *77* (1977), 581—85 an elegant method of coupling diethylenetriamine pentacetic acid with a protein, namely human serum albumin, as a result of which a useful chelating agent for metal ions, for example, radioactive indium, was obtained.

The radionuclide now most frequently used for radiodiagnostic purposes is technetium-99m which can easily become available in the location desired for use by elution of a molybdenum-technetium generator. Technetium-99m has a short half-life, namely 6 hours, and is a pure gamma emitter; as a result of this the radiation load for the patient is as small as possible. However, technetium-99m is not the best suitable radionuclide for all applications. For certain clinical uses, for example those which are described in a survey article by Goodwin c.s. (G. Subramanian (ed.), Radiopharmaceuticals, pp. 80—101, New York; Society of Nuclear Medicine (1975)), a radionuclide having a longer half-life is preferred. In nuclear medicine it is well-known, that labelled proteins generally circulate in the body for a long period of time and have a relatively slow clearance. Consequently the ratio of radioactive material in the target organ compared to that in the non-target organs remains low for a considerable time. In practice this may mean, that one has to wait for 24 to 48 hours after administration of the radioactive material before a usable scan can be made. For this application it is therefore desired to use proteins labelled with isotopes having longer half-lifes, preferably of a few days, viz. approximately the biological half-life of the protein. For this purpose best suitable in particular are compounds of indium-111, gallium-67, lead-203, ruthenium-97 and thallium-201. Of these compounds, compounds of indium-111 are most frequently used. Preferably between 2 and 3 days, but even up to 1 week after the administration of an indium-111 compound to a patient, the radiation emitted by the radioactive compound can excellently be recorded. Indium-111, having a half-life of approximately 67 hours, is a radioisotope which is produced in a cyclotron and is available as a chloride in 0.05 N hydrochloric acid. It is marketed carrier-free with an activity from 1 to 10 mCi per ml and a high radionuclidic purity. When just a radionuclide having a short half-life is desired, other isotopes, for example indium-113m or gallium-68, may be used.

Khaw and cooperators (Science *209* (1980), 295—297) have succeeded in coupling certain antibody fragments, so-called Fab fragments, with a derivative of diethylene triamine pentacetic acid, according to the method described by Krejcarek c.s. and have then converted the macromolecule, provided with chelating groups, with indium-111 chloride into a stable chelate. Khaw and cooperators used Fab fragments of anti-myosine antibodies as biological macromolecules and have demonstrated that after labelling these Fab fragments are potentially useful for localizing cardiac infarctions in vivo. However, the labelled Fab fragments of antibodies used by Khaw c.s. had in part lost their target organ specificity, so that radioactivity was also observed in the liver. According to Khaw c.s. in the same article, this is due to denaturation of the antibody fragments during the chelate formation with indium-111. As insufficient target organ specificity is very disadvantageous because correctly diagnosing is impeded by the radioactivity which is found in an organ or tissue other than the target organ or target tissue. This applies in particular when the organ deviation or the pathological process, for example a tumor, is still in an early stage of development; in fact, the timely detection of deviation or process can then be of life-saving importance. Moreover, an insufficient target organ specificity causes an undesired radiation load of organs other than the target organ.

For labelling macromolecules with radioactive indium the available radioactive indium cloride in aqueous solution is used. The highest pH at which this reaction can still be carried out successfully is a pH of 3.5. When it is desired to carry out this reaction in a more neutral medium, indium cannot be kept in solution. As a result of this the reaction does not occur, so that the macromolecular compound is not labelled. For compounds which are stable at a pH of 3.5, so in a comparatively acid medium, for example albumin, a reaction at a pH of 3.5 is, of course, not disadvantageous. However, many proteins, in particular antibodies and antibody fragments, are not sufficiently stable at a pH which is lower than the physiological pH, for example at a pH of 3.5 necessary to produce the chelating reaction with indium in the form of indium chloride.

This problem of the instability of various proteins in a comparatively acid medium is also mentioned in other publications, e.g. in the European patent application 0 038 546. So in this publication one has resorted to technetium-99m as the radionuclide, because "indium-111, and gallium-68, -67, require pH 3.5 for their solubility".

It is the object of the invention to provide a method of preparing radionuclide-labelled proteins which are insufficiently stable in acid medium, in particular antibodies or antibody fragments, by reacting a protein which is provided with chelating groups derived from desferrioxamine [1 - amino - 6,17 - dihydroxy - 7,10,28,21 - tetraoxo - 27(N - acetylhydroxy-amino) - 6,11,17,22 - tetraazaheptaeicosane], 8-hydroxyquinoline, iminotriacetic acid-containing chelating agents, or iminodiacetic acid-containing chelating agents, such as ethylene diamine tetracetic acid, diethylene triamine pentacetic acid or diaminocyclohexyltetracetic acid, or salts hereof, or a chelating peptide or polypeptide such as transferrine, with a solution of a radionuclide which in ion form is poorly soluble in an aqueous medium at neutral pH, without the protein denaturing during this reaction.

This object can be achieved by carrying out this reaction at a pH from 4 to 9, preferably from 6 to 7.5, with a solution comprising the radionuclide in the form of a complex, namely as an oxinate, a carboxylate, a dithiocarboxylate, an enolate, or a mixture thereof.

Suitable oxinates are formed with 8-hydroxyquinoline or derivatives hereof, suitable carboxylates are formed with di- or polycarboxylic acids or hydroxy carboxylic acids, such as oxalic acid, malonic acid, succinic acid, maleic acid, orthophthalic acid, malic acid, lactic acid, tartaric acid, citric acid, ascorbic acid, salicylic acid, iminodiacetic acid, iminotriacetic acid, or derivatives of any of these acids. Suitable dithio-carboxylates are dithiocarbamates, dithio-carbonates or derivatives hereof, and suitable enolates are formed with $\beta$-diketones, such as acetyl acetone, furoyl acetone, thenoyl acetone, benzoyl acetone, dibenzoyl methane, tropolone, pyrithione, or derivatives of any of these diketones.

As a matter of fact it has been found surprisingly that a solution of such a complex at a pH which is safe for a protein which is unstable in acid medium, for example, a physiological pH, is very suitable to convert the protein provided with chelating groups into the desired radioactive chelate, without the radionuclide in question precipitating from the solution and thus disturbing the reaction.

US patent 4,293,537 relates to the labelling of proteins with technetium-99m at physiological conditions. The pH of the reduced pertechnetate solution is adjusted to 7.4 with a solution of trisodium citrate and NaOH, a stable [99m]Tc-(Sn)citrate complex species being formed. As discussed before, technetium-99m is beyond the scope of the present invention, because this radionuclide is well soluble in an aqueous solution at neutral, e.g. physiological, pH. The initial reduction of pertechnetate, however, is generally carried out by stannous chloride in an acid medium. This is the reason that the technetium-99m containing solution requires a neutralization previous to the reaction with an acid-sensitive protein. Moreover, the technetium-99m complex posited in US 4,293,537 is brought into reaction with the pure protein. As discussed before, such a reaction has serious drawbacks. It is indeed surprising, that the reaction of the present invention, viz. between a solution of a complexed radionuclide, which in ion form is poorly soluble in an aqueous medium at neutral pH, and a protein which is provided with special chelating groups, proceeds so successfully, because in addition to a complexing agent and an acid-sensitive protein an extra chelating agent for the protein is needed, making the reaction medium, so subtile already, even more complicated.

Examples of radionuclides suitable for radiodiagnostic application and which in ion form at neutral pH are poorly soluble in aqueous medium are radioactive indium, radioactive gallium, lead-203, ruthenium-97, mercury-197 and thallium (III)-201. Because labelled macromolecules usually have a slow clearance, radionuclides having not too short a half-life are preferred, for example, indium-111, gallium-67, lead-203, ruthenium-97, mercury-197 and thallium(III)-201. The first four of these are to be preferred in connection with the suitable radiation characteristics and the availability; of these latter radionuclides, indium-111 is most current.

It is advantageous to carry out the chelating reaction with the radioactive indium compound at a physiological pH, because the solution of the labelled biological macromolecule formed can be administered to a patient as a radio-pharmaceutical composition without further treatment—not counting any desired sterilisation, for example, by means of millipore filtration.

A protein is preferably used which is provided with chelating groups derived from diethylene triamine pentacetic acid or salts hereof, because a suitable derivative of this chelating agent can very easily be coupled to the protein, for example according to the method described by Krejcareck c.s., a product being obtained which has excellent chelating properties for indium and in which the biological properties of the original protein have been well maintained.

The radionuclide is presented to the protein provided with chelating groups in the form of a complex with a chelating agent, namely as an oxinate, a carboxylate, a dithiocarboxylate, an enolate, or a mixture thereof. Examples of suitable chelating agents for the radionuclide are mentioned above. To be considered for this purpose are in particular an oxinate with 8-hydroxyquinoline, a carboxylate with citric acid or a derivative hereof, or an enolate with acetyl acetone as chelates, because it has been found that a complex of a radionuclide, for example indium-111, with any of these chelating agents in a suitable solvent, preferably in the form of a buffered aqueous solution, easily reacts at a suitable pH with a protein which is provided with chelating groups, preferably derived from diethylene triamine pentacetic acid, the desired radioactive chelate being formed in a satisfactory yield. A buffered aqueous indium-111-oxinate solution which is suitable for this purpose and which may be used to prepare the desired chelate is described in published Netherlands Patent Application No. 8007094. Such a solution as described in said published patent application can be prepared as follows. To 1 ml of water were added 1 mCi of carrier-free indium-111, chelated with 25 µg of 8-hydroxyquinoline, and sufficient sodium acetate buffer to adjust the pH at 3. If desired, the solution was made isotonic by the addition of a suitable amount of sodium chloride. Then before use sufficient sodium phosphate buffer was added to raise the pH to 6.5. Reference is made to the Examples for further indium-111-oxinate solutions.

As noted above, the resulting solution of the labelled protein may be used directly as a radio-pharmaceutical composition. If necessary, the solution may be brought into a form which is better suitable for intravenous or subcutaneous administration by a purification and/or by replacing the solvent by a liquid carrier which is better acceptable to the body, preferably physiological saline. Of course, it should be ensured that the protein does not denature during this treatment or these treatments. Of course, the solution must be sterile for intravenous or subcutaneous administration.

For carrying out a radiodiagnostic examination, a radiopharmaceutical composition which, in addition to a liquid carrier which is acceptable to the body, comprises radionuclide-labelled proteins, in particular antibodies or antibody fragments, if desired after dilution with a liquid which is acceptable to the body, for example physiological saline, may be administered to a living being in a quantity of $3.710^{-14}s^{-1}$ to $3.7.10^{-12}s^{-1}$ 100 µCi to 10 mCi), preferably from 1.85 to 11.1, $10^{-13}s^{-1}$ (0.5 to 3 mCi) per 70 kg of body weight, after which the radioactive radiation emitted by the living being is recorded.

The invention also relates to an equipment, a so-called "kit", for carrying out a radiodiagnostic examination which comprises (1) a protein which is provided with chelating groups derived from desferrioxamine, 8-hydroxyquinoline, iminotriacetic acid-containing chelating agents, or iminodiacetic acid-containing chelating agents such as ethylene diamine tetracetic acid, diethylene triamine pentacetic acid or diaminocyclohexyl tetracetic acid, or salts hereof, or a chelating peptide or polypeptide such as transferrine, whether or not in the dry state, (2) a solution which comprises a suitable radionuclide in the form of a complex, namely as an oxinate, a carboxylate, a dithiocarboxylate, an enolate, or a mixture thereof, and (3), if desired, directions for use with prescription for reacting (1) with (2).

In a preferred embodiment the "kit" comprises (1) a protein which is provided with chelating groups derived from diethylene triamine pentacetic acid or salts hereof, whether or not in the dry state, (2) a solution of a complex of a suitable radionuclide, preferably indium-111, gallium-67, lead-203, or ruthenium-97, with 8-hydroxyquinoline, acetyl acetone, citric acid or a derivative hereof, and (3), if desired, directions for use as stated above.

The component of the "kit" indicated above sub (1) may be present as a solution, for example in the form of a physiological saline solution, but also in the dry state, for example, in the lyophilized state.

The invention will now be described in greater detail with reference to the following specific examples.

Example I
Labelling Fab fragments of antibodies against human carcinoembryonic antigen (cea) with indium-111

In the usual manner, as described, for example, in Chinese Med. J. *23*, 237—41 (1976) antibodies against carcinoembryonic antigen (cea) were evoked in a goat. The antibodies were collected and purified by cea-Sepharose affinity chromatography of goat immune serum; Sepharose is a cellulose derivative. Purified antibodies were treated with papain for 1.5 hours at 37°C at a pH of 7.0 at an enzyme to substrate ratio of 1:100; as a result of this, Fab fragments were produced. Fab and Fc fragments were separated from each other by protein A-sepharose chromatography as a result of which the Fc fragments as well as indigested antibodies were bound. The Fab fragments of the antibodies were not bound to the column. In order to regenerate the column, the Fc fragments and the indigested antibodies were recovered with a 3 molar solution of guanidine hydrochloride in water. The Fab fragments of the antibodies were concentrated by vacuum dialysis and then dialyzed at a pH of 8.0 against a 0.1 molar solution of sodium bicarbonate. One millilitre of the concentrated solution of Fab fragments (5 mg/ml) were then reacted with carboxycarboxylic acid anhydride or diethylene triamine pentacetic acid (DTPA) in a 0.025 m molar solution in water, as described in the above-mentioned articles by Krejcarek c.s. and Khaw c.s. The resulting Fab fragments (DTPA fragments) provided with chelating groups were labelled with indium-111 in two manners, namely in the known manner at a pH of 3.5 and according to the method of the present invention at a pH of 7. The chelation reaction with indium-111 in the known manner at a pH of 3.5 was carried out by reacting an excess of indium-111 in the form of indium chloride at a pH of 3.5 with DTPA-Fab in a 0.1 molar solution of glycine hydrochloride in water and incubating the reaction mixture at room temperature for 30 minutes. The chelation reaction with indium-111 according to the invention at a pH of 7 was carried out by reacting DTPA-Fab with an excess of indium-111 in the form of an indium oxinate solution and incubating the reaction mixture at room temperature for 30 minutes. The indium-111-oxinate solution contained per millilitre 1 mCi of carrier-free indium-111, 25 µg of 8-hydroxyquinoline and 5.9 mg of sodium acetate. The pH of the indium oxinate solution had been adjusted at 7 right before use by adding to 1 ml of the solution 0.35 ml of a 0.2 molar buffer solution of tris(hydroxymethyl)aminomethane with a pH of 8.0. Both in case of the chelate (indium-111-DTPA-Fab) obtained in the known manner, and in case of the indium-111-DTPA-Fab produced at a pH of 7, free and chelated indium-111 were separated from each other by Sephadex® G-25 column chromatography (10 ml of column); Sephadex® is a cross-linked dextran. 0.15 Molar saline having a pH of 7.0 and buffered with 0.3 molar sodium phosphate was used as an eluent. The radioactive peaks in the void volume were dialysed against 2 litres of a 0.3 molar phosphate-buffered saline for 2 or 3 hours and then against a lactate-Ringer's solution for 6 to 12 hours. Both chelate solutions were then injected in nude mice bearing human colorectal carcinomas. There were three groups of 8 mice. In the first group, 0.3 ml of an indium-111-DTPA-Farb solution of $3.7—7.4 \cdot 10^{-14}s^{-1}$ (100—200 µCi) prepared at a pH of 3.5 was injected, in the second group 0.3 ml of the same solution, this time prepared at a pH of 7, and in the third group 0.3 ml of the same solution again prepared at a pH of 3.5 but now starting from Fab fragments obtained from antibodies evoked in a non-immunised goat. After 48 hours all the animals were sacrificed and tissue distribution studies were made. For that purpose, the radioactivity of the collected and weighed organs and tumors was measured in a Packard Autogamma scintillation counter; this counter comprises a NaI crystal. The radioactivity per gram of tissue was determined and the ratio of the activity in the tumor to that in the blood (tumor-blood ratio) was calculated. In the first group the average tumor-blood ratio was 8.6 (lowest value 4.2, highest value 12.1), in the second group 16.1 (lowest value 12.0 highest value 21.5), and in the third group 3.5 (lowest value 2.1, highest value 5.1). The third group which was injected with aspecific indium-111-DTPA-Fab was used as a control and enables the calculation of the specificity index. The specificity index is the ratio between the accumulated radioactivity in the target organ (target tissue) when specifically labelled antibodies are used, and the radioactivity in the same tissue when aspecific immunoglobulin is used. The specificity index for the indium-111-DTPA-Fab prepared at pH 3.5 is 8.6:3.5=2.5, that for the indium-111-DTPA-Fab prepared at pH 7 is 16.1:3.5=4.6.

From these experiments it has been found the specificity of indium-111-DTPA-Fab is improved by a factor 2 when the chelate is prepared at a pH of 7.

Example II
The experiments described in Example I were repeated, but now with indium-111-acetyl acetonate instead of indium-111-oxinate. The indium-111-acetyl acetonate solution contained per millilitre $3.7 \cdot 10^{-13}s^{-1}$ (1 mCi) carrier-free indium-111, 3 mg acetyl acetone and 5.9 mg sodium acetate. The pH of the indium-acetyl acetonate solution was adjusted at 7 before use by adding to 1 ml of the solution 0.35 ml of a 0.2 molar buffer solution of tris(hydroxymethyl)aminomethane with a pH of 8.0. All treatments were further the same as in Example I, so also the incubation of DTPA-Fab with indium-111-acetyl acetonate for 30 minutes at room temperature at a pH of 7, as well as the animal experiments with 8 nude mice with the chelate from DTPA-Fab and indium-111-acetyl acetonate produced at a pH of 7; the same control group was also used. An average tumor-blood ratio of 16.8 was found (lowest value 12.5, highest value 23.0). The specificity index for the indium-111-DTPA-Fab prepared by means of indium-111-acetylacetonate at a pH of 7 is 16.8:3.5=4.8.

From this experiment it appears that the same improvement of the specificity of indium-111-DTPA-Fab prepared at a pH of 7 is found, namely by a factor 2.

## Claims

1. A method of preparing radionuclide-labelled proteins which are unstable at a pH lower than the physiological pH, in particular antibodies or antibody fragments, by reacting a protein which is provided with chelating groups, derived from desferrioxamine, 8-hydroxyquinoline, iminotriacetic acid-containing chelating agents, or imino diacetic acid-containing chelating agents, such as ethylene diamine tetracetic acid, diethylene triamine pentacetic acid or diaminocyclohexyl tetracetic acid, or salts hereof, or a chelating peptide or polypeptide such as transferrine, with a solution of a compound of a radionuclide selected from radioactive indium, radioactive gallium, lead-203, ruthenium-97, mercury-197 and thallium-(III)-201, characterized in that the reaction is carried out at a pH from 4 to 9 with a solution of an oxinate, a carboxylate, a dithiocarboxylate, an enolate or a mixture thereof of the radionuclide.

2. A method as claimed in claim 1, characterized in that the radionuclide is used in the form of an oxinate with 8-hydroxyquinoline or a derivative hereof, a carboxylate with oxalic acid, malonic acid, succinic acid, maleic acid, orthophthalic acid, malic acid, lactic acid, tartaric acid, citric acid, ascorbic acid, salicylic acid, iminodiacetic acid, iminotriacetic acid, or a derivative of any of these acids, a dithiocarboxylate with a dithiocarbamic acid, a dithiocarboxylic acid, or a derivative hereof, or an enolate with acetyl acetate, furoyl acetone, thenoyl acetone, benzoyl acetone, dibenzoyl methane, tropolone, pyrithione, or a derivative of any of these diketones.

3. A method as claimed in claim 1 or 2, characterized in that a compound of indium-111, gallium-67, lead-203, or ruthenium-97 is used as a compound of a radionuclide.

4. A method as claimed in any of the preceding claims, characterized in that the reaction is carried out at a pH of 6 to 7.5, preferably at physiological pH.

5. A method as claimed in any of the preceding claims, characterized in that a protein is used which is provided with chelating groups derived from diethylene triamine pentacetic acid or salts hereof.

6. A method as claimed in any of the preceding claims, characterized in that the radionuclide is used in the form of a complex with 8-hydroxyquinoline, acetyl acetone, citric acid, or a derivative hereof.

7. Radionuclide-labelled proteins, in particular antibodies or antibody fragments, obtained by using the method as claimed in any of the preceding claims.

8. A radiopharmaceutical composition which, in addition to a liquid carrier material which is acceptable to the body, comprises radionuclide-labelled proteins, in particular antibodies or antibody fragments, characterized in that the composition comprises radionuclide-labelled proteins obtained by using the method as claimed in any of the claims 1—6.

9. A kit for performing a radiodiagnostic examination comprising (1) a protein which is provided with chelating groups derived from desferrioxamine, 8-hydroxyquinoline, iminotriacetic acid-containing chelating agent, or iminodiacetic acid-containing chelating agents such as ethylenediamine tetracetic acid, diethylenetriamine pentacetic acid or diaminocyclohexyl tetracetic acid, or salts hereof, or a chelating peptide or polypeptide such as transferrine, whether or not in the dry state, (2) a solution of an oxinate, a carboxylate, a dithiocarboxylate, an enolate or a mixture thereof, of a radionuclide selected from radioactive indium, radioactive gallium, lead-203, ruthenium-97, mercury-197 and thallium(III)-201, and (3), if desired, directions for use with a prescription for reacting (1) with (2).

10. A kit as claimed in claim 9, comprising (1) a protein which is provided with chelating groups derived from diethylenetriamine pentacetic acid, or salts hereof, whether or not in the dry state, (2) a solution of a complex of a radionuclide selected from indium-111, gallium-67, lead-203 and ruthenium-97 with 8-hydroxyquinoline, acetyl acetone, citric acid, or a derivative hereof, and (3), if desired, directions for use with a prescription for reacting (1) with (2).

## Ansprüche

1. Verfahren zum Herstellen von mit Radionukliden markierten Proteinen, die bei einem niedrigerem pH als dem physiologischen pH instabil sind, insbesondere Antikörpern oder Antikörperfragmenten, durch Umsetzen eines Proteins, das mit chelierenden Gruppen versehen ist, die von Desferrioxamin, 8-Hydroxychinolin, Iminotriessigsäure enthaltenden Chelierungsmitteln oder Iminodiessigsäure enthaltenden Chelierungsmitteln, wie Äthylendiamintetraessigsäure, Diäthylentriaminpentaessigsäure oder Diaminocyclohexyltetraessigsäure oder Salzen hievon, oder einem chelierenden Peptid oder Polypeptid, wie Transferrin, stammen, mit einer Lösung einer Verbindung eines Radionuklids ausgewählt aus radioaktivem Indium, radioaktivem Gallium, Blei-203, Ruthenium-97, Quecksilber-197 und Thallium(III)-201, dadurch gekennzeichnet, daß die Reaktion bei einem pH von 4 bis 9 mit einer Lösung eines Oxinats, eines Carboxylats, eines Dithiocarboxylats, eines Enolats oder einer Mischung hievon des Radionuklids durchgeführt wird.

2. Verfahren, wie in Anspruch 1 beansprucht, dadurch gekennzeichnet, daß das Radionuklid in Form eines Oxinats mit 8-Hydroxychinolin oder einem Derivat hievon, eines Carboxylats mit Oxalsäure, Malonsäure, Bernsteinsäure, Maleinsäure, Orthophthalsäure, Äpfelsäure, Milchsäure, Weinsäure, Citronensäure, Ascorbinsäure, Salicylsäure, Iminodiessigsäure, Iminotriessigsäure oder einem Derivat einer dieser Säuren, eines Dithiocarboxylats mit einer Dithiocarbaminsäure, einer Dithiocarbonsäure oder einem Derivat hievon oder eines Enolats mit Acetylaceton, Furoylaceton, Thenoylaceton, Benzoylaceton, Dibenzoylmethan, Tropolon, Pyrithion oder einem Derivat eines dieser Diketone verwendet wird.

3. Verfahren, wie in Anspruch 1 oder 2 beansprucht, dadurch gekennzeichnet, daß eine Verbindung von Indium-111, Gallium-67, Blei-203 oder Ruthenium-97 als eine Verbindung eines Radionuklids verwendet wird.

4. Verfahren, wie in einem der vorhergehenden Ansprüche beansprucht, dadurch gekennzeichnet, daß die Reaktion bei einem pH von 6 bis 7,5, vorzugsweise bei physiologischem pH, durchgeführt wird.

5. Verfahren, wie in einem der vorhergehenden Ansprüche beansprucht, dadurch gekennzeichnet, daß ein Protein verwendet wird, das mit chelierenden Gruppen versehen ist, die von Diäthylentriaminpentaessigsäure oder Salzen hievon stammen.

6. Verfahren, wie in einem der vorhergehenden Ansprüche beansprucht, dadurch gekennzeichnet, daß das Radionuklid in Form eines Komplexes mit 8-Hydroxychinolin, Acetylaceton, Citronensäure oder einem Derivat hievon verwendet wird.

7. Mit Radionukliden markierte Proteine, insbesondere Antikörper oder Antikörperfragmente, erhalten durch Anwendung des Verfahrens, wie in einem der vorhergehenden Ansprüche beansprucht.

8. Radiopharmazeutische Zusammensetzung, die außer einem flüssigen Trägermaterial, das für den Körper annehmbar ist, mit Radionukliden markierte Proteine, insbesondere Antikörper oder Antikörperfragmente aufweist, dadurch gekennzeichnet, daß die Zusammensetzung mit Radionukliden markierte Proteine aufweist, die durch Anwendung des Verfahrens, wie in einem der Ansprüche 1 bis 6 beansprucht, erhalten wurden.

9. Kit zum Durchführen einer radiodiagnostischen Untersuchung, umfassend (1) ein Protein, das mit chelierenden Gruppen versehen ist, die von Desferrioxamin, 8-Hydroxychinolin, Iminotriessigsäure enthaltenden Chelierungsmitteln oder Iminodiessigsäure enthaltenden Chelierungsmitteln, wie Äthylendiamintetraessigsäure, Diäthylentriaminpentaessigsäure oder Diaminocyclohexyltetraessigsäure oder Salzen hievon oder einem chelierenden Peptid oder Polypeptid, wie Transferrin, stammen, egal ob im trockenen Zustand oder nicht, (2) eine Lösung eines Oxinats, eines Carboxylats, eines Dithiocarboxylats, eines Enolats oder einer Mischung hievon eines Radionuklids ausgewählt aus radioaktivem Indium, radioaktivem Gallium, Blei-203, Ruthenium-97, Quecksilber-197 und Thallium(III)-201 und (3) wenn gewünscht Anweisungen zur Verwendung mit einer Vorschrift zum Umsetzen von (1) mit (2).

10. Kit, wie in Anspruch 9, beansprucht, umfassend (1) ein Protein, das mit chelierenden Gruppen versehen ist, die von Diäthylentriaminpentaessigsäure oder Salzen hievon stammen, egal ob in trockenem Zustand oder nicht, (2) eine Lösung eines Komplexes eines Radionuklids ausgewählt aus Indium-111, Gallium-67, Blei-203 und Ruthenium-97 mit 8-Hydroxychinolin, Acetylaceton, Citronensäure oder einem Derivat hievon, und (3) wenn gewünscht, Anweisungen zur Verwendung mit einer Vorschrift zum Umsetzen von (1) mit (2).

**Revendications**

1. Procédé de préparation de protéines marquées par un radionucléide, qui sont instables à un pH inférieur au pH physiologique, en particulier d'anticorps ou de fragments d'anticorps, par réaction d'une protéine qui est munie de groupes de chélation, dérivés de la déféroxamine, de la 8-hydroxyquinoléine, d'agents de chélation contenant de l'acide iminotriacétique ou d'agents de chélation contenant de l'acide iminodiacétique, comme l'acide éthylène diamine tétracétique, l'acide diéthylène triamine pentacétique ou l'acide diaminocyclohexyl tétracétique, ou leurs sels, ou un peptide ou un polypeptide chélateur comme la transferrine, avec une solution d'un composé d'un radionucléide choisi parmi l'indium radioactif, le gallium radioactif, le plomb-203, le ruthénium-97, le mercure-197 et le thallium(III)-201, caractérisé en ce qu'on effectue la réaction à un pH de 4 à 9 avec une solution d'oxynate, d'un carboxylate, d'un dithiocarboxylate, d'un énolate ou un de leurs mélanges, du radionucléide.

2. Procédé selon la revendication 1, caractérisé en ce qu'on utilise le radionucléide sous forme d'un oxinate formé avec la 8-hydroxyquinoléine ou un de ses dérivés, d'un carboxylate formé avec l'acide oxalique, l'acide malonique, l'acide succinique, l'acide maléique, l'acide orthophtalique, l'acide malique, l'acide lactique, l'acide tartrique, l'acide citrique, l'acide ascorbique, l'acide salicylique, l'acide iminodiacétique, l'acide iminotriacétique ou un dérivé de l'un quelconque de ces acides, d'un dithiocarboxylate formé avec un acide dithiocarbamique, un acide dithiocarboxylique ou un de leurs dérivés, ou d'un énolate formé avec l'acétyl acétone, la furoyl acétone, la thénoyl acétone, la benzoyl acétone, le dibenzoyl méthane, la tropolone, la pyrithione, ou un dérivé de l'une quelconque de ces dicétones.

3. Procédé selon la revendication 1 ou 2, caractérisé en ce qu'on utilise un composé de l'indium-111, du gallium-67, du plomb-203, ou du ruthénium-97 à titre de composé d'un radionucléide.

4. Procédé selon l'une quelconque des revendications précédentes, caractérisé en ce qu'on effectue la réaction à un pH de 6 à 7,5, de préférence au pH physiologique.

5. Procédé selon l'une quelconque des revendications précédentes, caractérisé en ce qu'on utilise une protéine qui est munie de groupes de chélation dérivés de l'acide diéthylène triamine pentacétique ou ses sels.

6. Procédé selon l'une quelconque des revendications précédentes, caractérisé en ce qu'on utilise le radionucléide sous forme d'un complexe formé avec la 8-hydroxyquinoléine, l'acétyl acétone, l'acide citrique ou un de leurs dérivés.

7. Protéines, en particules anticorps ou fragments d'anticorps, marquées par un radionucléide, obtenues par utilisation d'un procédé selon l'une quelconque des revendications précédentes.

8. Composition radiopharmaceutique qui, en plus d'un véhicule liquide qui est acceptable pour l'organisme, comprend des protéines, en particuler des anticorps ou fragments d'anticorps, marquées par un radionucléide, caractérisée en ce qu'elle comprend des protéines marquées par un radionucléide que l'on obtient en utilisant le procédé selon l'une quelconque des revendications 1 à 6.

9. Nécessaire pour effectuer un examen par radiodiagnostic, comprenant (1) une protéine munie de groupes de chélation dérivés de la déféroxamine, de la 8-hydroxyquinoléine, d'agents de chélation contenant de l'acide iminotriacétique, ou d'agents de chélation contenant de l'acide iminodiacétique comme l'acide éthylène diamine tétracétique, l'acide diéthylène triamine pentacétique ou l'acide diaminocyclohexyltétracétique ou leurs sels, ou un peptide ou un polypeptide chélateur comme la transferrine, à l'état sec ou non, (2) une solution d'un oxinate, d'un carboxylate, d'un dithiocarboxylate, d'un énolate ou d'un de leurs mélanges, d'un radionucléide choisi parmi l'indium radioactif, le gallium radioactif, le plomb-203, le ruthénium-97, le mercure-197 et le thallium(III)-201, et (3), si on le désire, un mode d'emploi avec une prescription pour faire réagir (1) avec (2).

10. Nécessaire selon la revendication 9, comprenant (1) une protéine munie de groupes de chélation dérivés de l'acide diéthylène triamine pentacétique, ou ses sels, à l'état sec ou non, (2), une solution d'un complexe d'un radionucléide choisi parmi l'indium-111, le gallium-67, le plomb-203 et le ruthénium-97 avec la 8-hydroxyquinoléine, l'acétyl acétone, l'acide citrique ou un de leurs dérivés, et (3) si on le désire, un mode d'emploi avec une prescription pour faire réagir (1) avec (2).